# FASCICULE DE BREVET EUROPEEN

(11) **EP 0 407 409 B1**
(45) Date de publication et mention de la délivrance du brevet: **15.06.1994**
(21) Numéro de dépôt: 89903181.9
(22) Date de dépôt: 03.03.1989
(51) Int. Cl.: A61B 8/08, A61F 2/30

(54) **PROCEDE DE REPRODUCTION DE RELIEF OSSEUX PAR ECHOGRAPHIE ULTRASONORE TRIDIMENSIONNELLE**
VERFAHREN ZUR WIEDERGABE DER UMRISSE VON KNOCHEN MITTELS ULTRASCHALL-ECHOGRAPHIE
PROCESS FOR REPRODUCING A BONE RELIEF BY TRIDIMENSIONAL ULTRASOUND ECHOGRAPHY

(30) Priorité: 04.03.1988 FR 8802996
(43) Date de publication de la demande: 16.01.1991
(73) Titulaire: CESSOT, Christian, F-13007 Marseille (FR)
(72) Inventeur: CESSOT, Christian, F-13007 Marseille (FR)
(74) Mandataire: Roman, Michel
(86) Numéro de dépôt international: FR8900088
(87) Numéro de publication internationale: WO8907910

(56) Documents cités:
- EP-A- 0 255 797
- DE-A- 3 719 919
- FR-A- 2 577 697

## Description

L'objet de l'invention se rapporte à un procédé de reproduction de relief osseux par échographie ultrasonore tridimensionnelle.

Il est destiné à la réalisation d'empreintes osseuses, sans intervention chirurgicale, notamment en art dentaire, et plus particulièrement en implantologie, ou cette technique permet de réaliser des implants "Juxta-osseux" en supprimant l'empreinte sanglante de l'os, grâce à l'obtention d'une image en trois dimensions par la visualisation des reliefs osseux au travers des teguments et des muscles au moyen de sondes ultrasonores.

Actuellement, lorsque l'indication d'un implant osseux a été posée, il est; obligatoire de réaliser dans un premier temps une intervention qui permet de prendre l'empreinte de l'os du site receveur.

Cette intervention nécessite une incision et un décollement très large des tissus et des insertions musculaires de tout le site implantaire. Une fois l'empreinte prise, les tissus sont remis en place et suturés.

La deuxième phase est la réalisation au laboratoire de l'implant:
- Coulée en plâtre de l'empreinte, ce qui donne un modèle positif de l'os du site implantaire;
- Réalisation, sur ce modèle, de la maquette en cire de l'implant;
- Coulée del'implant en Cr-Co par la méthode de la cire perdue;
- Grattage et polissage de l'implant.

Cet implant doit alors être posé lors d'une deuxième intervention, soit le même jour, deux à trois heures plus tard si le laboratoire est sur place, soit quelques jours plus tard.

Des méthodes ont été également recherchées pour reconstituer un relief osseux, mais au moyen de scanners à rayons X, qui sont beaucoup plus dangereux pour le corps humain que les ultrasons. De plus, les scanners à rayons X actuels sont très volumineux et très onéreux. Une telle méthode est présentée dans le brevet EP-A-0 255 797, mais elle n'expose en fait qu'un moyen de reconstituer un os détruit en partant de l'os correspondant symétrique du patient et l'utilisation de tomographie informatique tridimensionnelle à rayons X.

Le brevet FR-A-2 577 697 expose un moyen de déterminer la forme d'un implant endo-osseux destiné aux canaux médullaires d'os longs grâce a l'utilisation d'un scaner tomographique à rayons X ou à résonance magnétique. Cependant, ce moyen n'est absolument pas adapté à la réalisation d'implants "juxta-osseux".

Il faut remarquer que l'utilisation des rayons X ou de la résonance magnétique ne permet pas de mesurer l'épaisseur des tissus mous.

Le brevet N^{o} DE-A-3 719 919 décrit un appareil d'exploration tomographique à ultrasons destiné au diagnostique en particulier de l'os de la hanche. Cet appareil permet de générer une image de l'os, mais n'est pas utilisable pour la réalisation d'empreinte osseuse, en particulier dans le domaine de l'implantologie dentaire.

Le procédé selon l'invention remédie à tous ces inconvénients. En effet, il permet d'éviter la première intervention chirurgicale qui est extrêmement traumatisante, et il utilise pour obtenir une image du relief osseux un appareillage beaucoup plus petit, et beaucoup moins onéreux, comparativement aux systèmes utilisant des rayons X.

Il est constitué par la combinaison d'abord d'un générateur-récepteur équipé d'une sonde à ultrasons, ponctuelle ou multi-points, fixe ou mobile, destinés à mesurer par échographie, l'épaisseur des tissus mous qui recouvrent une zone osseuse, ensuite d'un ordinateur relié au générateur-récepteur permettant de stocker les mesures et de visualiser le relief osseux en trois dimensions sur un écran moniteur, enfin d'une machine-outil programmable qui va diminuer un modèle en plâtre de la zone étudiée, de l'épaisseur correspondante des tissus mous, et donner ainsi un modèle en plâtre du relief osseux sousjacent.

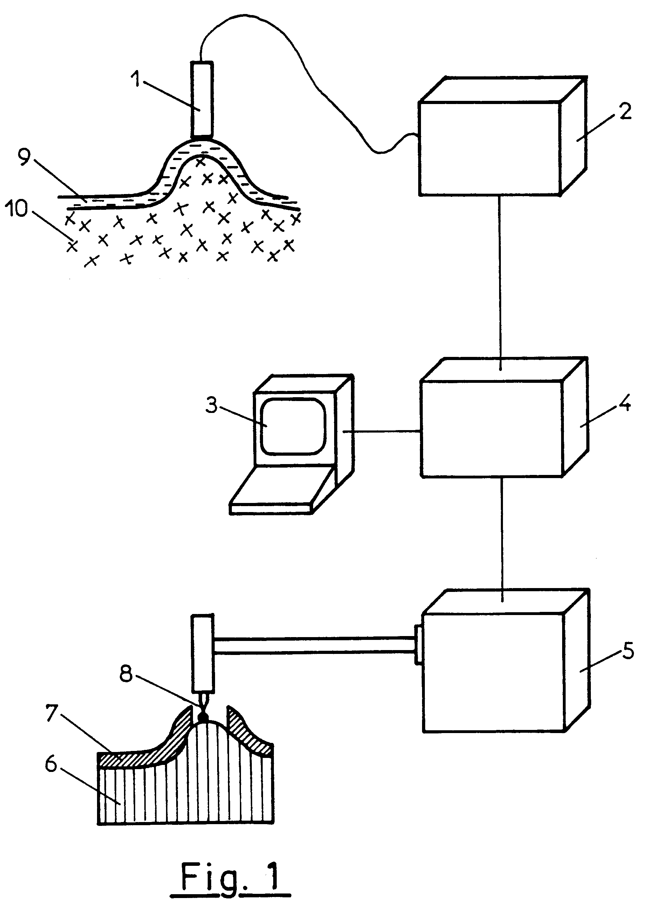

Le dessin annexé, donné à titre d'exemple non limitatif d'une des forme de l'invention, illustre un mode de réalisation du procédé conforme à la présente invention.

La réalisation d'un implant juxta-osseux s'effectue de la manière suivante:
- Prise d'une empreinte du site sans recliner les tissus mous 9. (Coulée en plâtre pour faire le modèle positif 6).
- Détermination en bouche de l'épaisseur des tissus 9 recouvrant le site 10, sans effraction chirurgicale, par échographie tridimensionnelle, au moyen de la sonde ultrasonore 1, reliée au générateur-récepteur 2.
- conversion analogique-numérique des mesures effectuée par l'ordinateur 4 relié au générateur 2.
- traîtement et stockage des données par l'ordinateur et visualisation sur le moniteur 3, d'une image de contrôle en trois dimensions du site 10.
- Elaboration, par l'ordinateur 4, d'un programme (ou d'un fichier) destiné au contrôle de la machine-outil 5, ou pilotage direct de ladite machine-outil par l'ordinateur, en fonction des données reçues de la sonde ultrasonore 1 dûment traîtées.
- Décortication du modèle en plâtre 6, de l'épaisseur correspondante 7 de tissus mous en chaque point du modèle, à l'aide d'une machine-outil programable 5 équipée d'une fraise 8, afin d'obtenir un modèle 6 de l'os sous-jacent 10.

La partie restante 6 correspond dès lors exactement à ce qui aurait été obtenu en prenant une empreinte par coulage directement sur l'os, après incision chirurgicale.
- Réalisation de l'implant en laboratoire sur ce modèle ainsi corrigé.
- Enfin, mise en place de l'implant, de la maniere habituelle.

Ce schéma de réalisation comporte deux problèmes techniques essentiels à résoudre:
1°. Comment déterminer l'épaisseur des tissus mous ?
2°. Comment faire décortiquer le modèle de plâtre de cette épaisseur ?

Le deuxième point est résolu par l'utilisation d'une machine-outil à commande numérique adaptée à ce genre de travail.

Les techniques d'asservissement d'une telle machine à partir de mesures traîtées par ordinateur sont actuellement bien maîtrisées et s'appliqueront sans difficultés. Il en va de même pour la programmation proprement dite de l'ordinateur, aussi bien pour la visualisation que pour le pilotage de la machine-outil.

Le point essentiel est la mesure de l'épaisseur des tissus mous. Pour y parvenir, il est fait appel à un dispositif utilisant des sondes à ultrasons ponctuelles, ou à barrettes, ou encore à balayage sectoriel, suivant les cas.

L'échographie ultrasonore convient très bien à ce type d'application. En effet, les interfaces entre les os et les tissus mous donnent des images particulièrement nettes, surtout à faible profondeur. En fonction du résultat à obtenir, on pourra avoir recours à un balayage manuel, mécanique motorisé ou éléctronique dans le cas de sondes équipées de nombreux transducteurs.

Grâce à sa relative simplicité, son volume réduit et son coût de revient raisonnable, le procédé qui vient d'être décrit se prête bien à la réalisation d'installations de prise d'empreintes pour la confection d'implants dentaires. De telles installations pourraient être utilisées conjointement pour le diagnostic de certaines affections osseuses.

Les principes énoncés peuvent également s'appliquer dans d'autres domaines médicaux, en particulier chaque fois qu'il est nécessaire de connaître de façon précise la structure d'une surface osseuse.

Le positionnement des divers éléments constitutifs donnent à ce dispositif un maximum d'effets utiles qui n'avaient pas été obtenus a ce jour par des dispositifs similaires.

Cependant, les formes, dimensions et dispositions des différents éléments, ainsi que les types d'appareils utilisés, pourront varier dans la limite des équivalents, sans changer pour cela, la conception générale de l'invention qui vient d'être décrite.

## Revendications

1. Procédé de reproduction de relief osseux destiné à la réalisation d'empreintes osseuses, sans intervention chirurgicale, notamment en implantologie dentaire, et pour la réalisation d'implants "Juxta-osseux",
caractérisé d'une part, par l'utilisation de l'échographie ultrasonore tridimensionnelle, au moyen d'une sonde ultrasonore (1) associée à un générateur-récepteur d'ultrasons (2), lui-même relié à un ordinateur (4) équipé d'un écran de visualisation (3), et d'une machine-outil (5) à commande numérique, comportant un moyen de fraisage (8), pilotée par l'ordinateur (4), directement ou par l'intermédiaire de fichiers enregistrés, d'autre part par le déroulement des opérations selon les phases suivantes:
a. Prise d'une empreinte des tissus mous (9) recouvrant le site osseux (10) et coulage dans cette empreinte d'un modèle positif (6) en plâtre;
b. Détermination en tout point de l'épaisseur des tissus mous (9) recouvrant le site osseux (10) par échographie tridimensionnelle, au moyen d'une sonde ultrasonore (1) reliée au générateur-récepteur (2), ladite sonde étant de type ponctuel ou à plusieurs éléments émetteurs-récepteurs, utilisant un balayage manuel, mécanique motorisé ou éléctronique dans le cas de sondes équipées de nombreux transducteurs;
c. Conversion analogique-numérique des mesures par l'ordinateur (4) relié au générateur-récepteur d'ultrasons (2):
d. Elaboration, par l'ordinateur (4), d'un programme (ou d'un fichier) destiné au contrôle de la machine-outil (5), ou pilotage direct de ladite machine-outil par l'ordinateur, en fonction des données reçues de la sonde ultrasonore (1);
e. Décortication du modèle positif (6), de l'épaisseur correspondante (7) des tissus mous en chaque point du modèle, à l'aide de la machine-outil (5) équipée d'une fraise (8), afin d'obtenir un modèle du site osseux (10) sous-jacent, la partie restante du modèle positif (6) correspondant dès lors exactement à ce qui aurait été obtenu en prenant une empreinte par coulage directement sur le site osseux (10), après incision chirurgicale.

2. Procédé suivant la revendication 1, se caractérisant par l'utilisation d'une sonde échographique (1) de type à barrettes, à balayage sectoriel, pour la réalisation d'implants dentaires juxta-osseux.

## Claims

1. Process for reproducing bone reliefs for making bone prints without surgical intervention, particularly in dental implantology and for "Juxta-osseux" implants,
characterized on the one hand by the use of three-dimensional ultrasonic echography using an ultrasonic probe (1) associated with an ultrasonic receptor/generator (2), itself connected to a computer (4) equipped with display screen (3) and a numerically controlled machine tool (5) with a mill tool (8) controlled by the computer (4), directly or through recorded files, and secondly by sequences of operation which use the following phases:
a. Prints of soft tissu (9) covering the bone area (10) and pouring a positive plaster model (6) into this print;
b. Determination of the thickness of sort tissu (9) at all points covering bone area (10) by three-dimensional echography using ultrasonic probe (1) connected to generator/receiver (2), the said probe being of the instantaneous type or with several emitter/receiver elements, using manual, motorized mechanical or electronic sweeping in the case of a probe equipped with many transducers;
c. Alphanumeric conversion of readings by the computer (4) connected to the ultrason generator/receiver (2);
d. Generation by the computer (4) of a programme (or a file) for controlling the machine tool (5), or direct pilotting of the said machine tool by the computer in relation to the data received from the ultrasonic probe (1);
e. Decortication of the positive model (6), of corresponding thickness (7) to the soft tissu at each point of the model, using the machine-tool (5) equipped with mill (8) in order to obtain a model of the underlying bone area (10), the remaining part of the positive model (6) then corresponding exactly to that which would have been obtained by taking a print by direct pouring on the bony area (10) after surgical incision.

2. Process as per claim 1 characterized by the use of a bar-type echographic probe (1), with sectorial sweep, for the execution of "juxta-osseux" dental implants.

## Patentansprüche

1. Verfahren zur Reproduktion des Knochenreliefs, bestimmt zur Herstellung von Knochenabformungen ohne chirurgischen Eingriff, insbesondere in der Zahnimplantation, und zur Verwirklichung von "knochenanliegenden" Implantaten,
gekennzeichnet einerseits durch die Verwendung der dreidimensionalen, überhörfrequenten Echographie mit Hilfe einer Ultraschallsonde (1) in Verbindung mit einem Ultraschall-Generator/Empfänger (2), der seinerseits an einen mit einem Bildschirm (3) ausgestatteten Computer (4) angeschlossen ist, und einer Werkzeugmaschine (5) mit numerischer Steuerung, umfassend ein Fräswerkzeug (8), direkt oder mittels aufgezeichneter Dateien gesteuert durch den Computer (4), und andererseits durch den Ablauf der Vorgänge entsprechend den folgenden Phasen:
a. Aufzeichnung eines Abdrucks der die Knochenstelle (10) überlagernden weichen Gewebe (9) und Gießen in diesem Abdruck eines positiven Gipsmodells;
b. Bestimmung an allen Punkten der Tiefe der weichen Gewebe (9) über der Knochenstelle (10) durch dreidimensionale Echographie, mittels der mit dem Generator/Empfänger (2) verbundenen Ultraschallsonde (1), wobei die besagte Sonde punktueller Art ist oder mehrere Sender/Empfängerelemente besitzt, mit manueller, mechanisch-motorisierter oder, im Fall von mit zahlreichen Meßwandlern ausgerüsteten Sonden, elektronischer Abtastung;
c. Analog-digitale Konvertierung der Meßwerte durch den an den Ultraschall-Generator/Empfänger (2) angeschlossenen Computer (4);
d. Erarbeiten durch den Computer (4) eines Programms (oder einer Datei), bestimmt zur Kontrolle der Werkzeugmaschine (5) oder direkte Steuerung der besagten Werkzeugmaschine durch den Computer in Abhängigkeit der von der Ultraschallsonde (1) erhaltenen Daten;
e. Entschalen der den weichen Geweben entsprechenden Dicke (7) des Positiven Modells (6) an allen seinen Punkten mittels der mit einer Fräse (8) ausgerüsteten Werkzeugmaschine (5), um ein Modell der darunter liegenden Knochenstelle (10) zu erhalten, wobei der restliche Teil des positiven Modells genau dem entspricht, was man nach chirurgischem Einschnitt mit einem direkten Abguß auf der Knochenstelle (10) erhalten hätte.

2. Verfahren gemäß Anspruch 1, gekennzeichnet durch die Verwendung einer echographischen Sonde (1) des Barretten-Types, mit sektorieller Abtastung, zur Verwirklichung von knochenanliegenden Zahnimplantaten.
